# EUROPEAN PATENT SPECIFICATION

(11) **EP 4 232 137 B1**
(45) Date of publication and mention of the grant of the patent: **02.07.2025**
(21) Application number: 21790924.1
(22) Date of filing: 15.10.2021
(51) Int. Cl.: A61N 1/05

(54) **LEAD ANCHOR FOR A NEUROMODULATION LEAD**
ANKER FÜR EINE NEUROMODULATIONSLEITUNG
ANCRANGE POUR CONDUCTEUR DE NEUROMODULATION

(30) Priority: 22.10.2020 US 202063104074 P; 19.02.2021 EP 21158027
(43) Date of publication of application: 30.08.2023
(73) Proprietor: BIOTRONIK SE & Co. KG, 12359 Berlin (DE)
(72) Inventor: MELIUS, Matthew, Portland, OR 97206 (US); BECKER, Andreas, Tigard, OR 97224 (US); AUSTIN, Eric, Portland, OR 97221 (US); KIBLER, Andrew, B., Lake Oswego, OR 97035 (US)
(74) Representative: Biotronik Corporate Services SE
(86) International application number: PCT/EP2021/078591
(87) International publication number: WO 2022/084177

(56) References cited:
- EP-A1- 2 902 074
- EP-A1- 3 511 050
- WO-A1-2013/112920
- US-A1- 2010 274 336
- US-A1- 2015 045 865
- US-A1- 2020 108 247

## Description

The present invention is generally directed to a lead anchor for a neuromodulation lead that can be used for spinal cord stimulation of a patient in order to relieve pain by electrically activating pain-inhibiting neurons in the posterior horn. Such a lead anchor may comprise a solid anchor block having at least one opening and a lumen configured to receive a portion of a neuromodulation lead. Further, the lead anchor is configured to fix the neuromodulation lead at the patient's body.

Spinal cord stimulation (SCS) is an emerging technique for providing long-term pain relief to a patient as an alternative to medication, in the case where corrective surgery is no longer an option. The effectiveness of SCS depends on the positioning of two sets of electrodes at the end of conductive leads within the spinal column. The position of the electrodes relative to each other and to the spinal cord is crucial for the success of the therapy. To ensure that the electrodes do not move after placement, a lead anchor is placed along the electrodes at the point where they enter the spinal column for fixing the electrode leads.

Conventionally, a lead anchor most often comprises a silicone anchor body which has a passage such as a hollow cylindrical portion where the lead is passed through. **In** addition, the lead anchor often has at least one eyelet to suture the lead anchor into the patient's body and hold it in position. The lead anchor is typically able to hold the lead in a relatively secure position. However, the lead anchor has some disadvantages with regard to axial forces. It is therefore not surprising that axial forces acting on the lead anchor and/or the lead are the most common cause of lead migration in SCS. For this reason, it is necessary to provide a fixing mechanism that avoids axial movement between the lead anchor and the neuromodulation lead occurs.

Based on the preference expressed by specialists, a set screw with threaded shaft and a torque wrench, which are commonly used for connecting leads to implantable pulse generators, are desirable to fix the lead within the lead anchor.

A possible implementation for such a lead anchor is disclosed in US 2020/0108247 A1. Here, the lead anchor comprises an anchor body with a passage which is configured to receive a lead. Further, the lead anchor comprises a fixing mechanism which serves to fix a lead inserted into the lead anchor and to protect the lead from axial displacement. A screw within said fixing mechanism may activate the fixing mechanism so that a force is applied to said lead. In addition, the lead anchor comprises eyelets for suturing the lead anchor inside the patient's body.

Regardless of whether a screw is in direct contact with the lead or activates a fixing mechanism, the difficulty with such a configuration is not to close the fixing mechanism too tightly, as this could damage the lead. On the other hand, if the screw is too loose, the lead will not be properly secured, and the lead may (axially) slip within the anchor body.

To avoid these drawbacks, the lead anchor disclosed in US 2020/0108247 A1 provides a mesh for force distribution across the lead. However, the screw may still be overtightened so that the force applied to the lead by the fixing mechanism may damage the lead. In the same way, the screw can also be fastened too loosely, causing the lead to slip.

Depending on when such lead damage and/or slippage is discovered, in the worst case, the patient may need to undergo surgery again and a full lead replacement of the lead and/or lead anchor may be required. Since each surgery involves a risk for the patient in addition to the costs, it should be avoided, if possible.

Further prior art is known from WO 2013/112920 A1, US 2015/045865 A1, EP 2902074 A1,EP 3511050 A1 and US 2010/274336 A1.

A further drawback of known lead anchor system is the retention of the screw in the lead anchor. Conventionally, the screw is covered by the flexible silicone of the anchor body. It was observed that at the upper end of the screw, the actual holding force is relatively low, which may lead to accidental removal.

The access to the tool mount of the screw is another drawback of the known lead anchor system. As mentioned above, the screw is covered by flexible silicone. The tool mount may also be covered, so that due to the small size of the tool mount of the screw, usually M2 screws are used, and due to a relatively short tool engagement length, the application is quite complicated and user unfriendly. This is mainly due to the lack of visual feedback for the implanting physician to confirm that the torque wrench is fully engaged in the tool mount before applying torque to fix the lead by the fixing element. The lack of visual feedback leads to uncertainty and reduced confidence in the procedure, which increases the time required for implantation and potentially increases the risk for the patient.

Another drawback of known lead anchors in the art refers to the fixing inside the patient's body. Usually eyelets configured for fastening the anchor body to the tissue of a patient, e.g. by means of a suture (commonly referred to as 'suture loops') are used, wherein the eyelets are formed from silicone of the anchor body. Although the flexible silicone is desirable for patient comfort, it can be easily damaged or even teared completely, thus impairing or destroying the suture's fixation function. This may occur during implantation, or the flexible suture loops may deteriorate over time due to patient's movement. **In** addition, the flexible silicone suture loops may result in an insecure and instable position of the lead anchor within the patient's body.

Therefore, it is an object of the present invention to provide a lead anchor for a neuromodulation lead, especially for SCS, which provides an improved fixing mechanism of the lead, wherein the fixing mechanism prevents, for example, any damage or slippage of the lead. Further, it is an object of the present invention to provide a fixing mechanism preventing accidental removal of a fixing element. Additionally, an object of the present invention is to improve the fixing of the lead anchor within the patient's body. Further, it is an object of the present invention to provide a simple method for manufacturing said lead anchor having favorable manufacturing costs.

All the above-mentioned objects pursue the aim of making the implanted lead anchor more robust against axially acting forces, so that primarily the object of the present invention is to prevent slippage of the lead and/or slippage of the lead anchor.

The object is solved by a lead anchor for a neuromodulation lead with the features of claim 1. Further, the object is solved by a method for manufacturing of such lead anchor according to claim 12 as well.

A lead anchor for a neuromodulation lead according to the present invention comprises a solid anchor block having at least one opening and a lumen. The lumen is configured to receive a portion of the neuromodulation lead. Further, the at least one opening of the anchor block is connected at one end (lower end) with said lumen and has a longitudinal axis. In addition, the lead anchor comprises an anchor body, at least partially covering said anchor block, and a fixing element. The fixing element has a fastening portion and a head portion, wherein said fastening portion is configured to be inserted along the longitudinal axis into the opening to fix the portion of the neuromodulation lead. The head portion comprises at least one first stop feature having a dimension in a radial direction with respect to said longitudinal axis which is greater than a diameter of said opening.

The fact that the anchor block is 'solid' means that the anchor block has an increased material stiffness compared to a soft material. In relation to the lead anchor, 'solid' means that the anchor block has a higher material stiffness than the anchor body. Further, the term 'solid' may be understood in relation to the configuration of the anchor block in that the anchor block is formed from a solid material or has a corresponding geometry that favors the stiffness of the anchor block compared to the anchor body. In one embodiment the anchor block has an essential cylindrical form and provides a stable core within the lead anchor in which the lead is effectively fixed. The anchor body provides protection of the anchor block and a comfortable seat of the lead anchor at the patient's body.

The at least one opening within the anchor block serves to guide one fixing element. The number of openings in the anchor block may match the number of fixing elements, so that one opening receives one fixing element. In addition, each opening comprises a longitudinal axis, which may be perpendicular to the opening cross-section. The longitudinal axis may cross the center of the opening cross-section and runs along the longitudinal extension of the opening. Furthermore, a fixing element may be inserted into the opening along this longitudinal axis. The lumen of the anchor block may be straight or curved. However, the opening always connects to the lumen in such a way, that if a fixing element is sufficiently inserted into said opening, the neuromodulation lead will be fixed. Thus, the lumen may be cylindrical comprising a longitudinal axis. The opening may be arranged such that its longitudinal axis is perpendicular to the longitudinal axis of the lumen or inclined to it. The opening may have a cylindrical form, as well.

The fixing element comprises a fastening portion and a head portion. With the fastening portion the fixing element interacts with the opening to secure the fixing element with regard to the anchor block. Accordingly, the opening comprises engagement means to secure the fixing element and to interact with respective engagement means of the fastening portion. The fixing element may be realized by a screw having a flange-like projection at its upper end or by a cap screw. The fastening portion is formed in both cases by a thread along the shaft of the screw and the head portion by the upper end of the screw with the flange-like projection or by the screw head which does not have any fastening means. If the fixing element is realized by a screw, the opening also comprises a respective threaded portion in order to receive and guide the thread of the fastening portion of the screw. Furthermore, the screw head (or cap), provides the first stop feature by one face of the head running perpendicular to its longitudinal axis. The first stop feature may alternatively formed by a face of the flange-like projection. Further alternatively, the fixing element may have a head portion as described above and a fastening portion with an engagement mechanism other than a thread, for example, at least one pin-line projection at the surface of the fastening portion that engages with a respective, e.g. helical groove at the inner surface of the opening.

According to the invention, the dimension of the first stop feature in the radial direction is greater than the diameter of the opening. The term 'diameter' is intended to refer to the area of a cross section of the opening, wherein the cross section is perpendicular to its longitudinal axis. The diameter is a maximum dimension across this cross section, e.g. a diameter of a circular cross section. **In** the case of a square opening, the diameter of the opening would thus be formed by the diagonal line of the square. **In** the case of a hexagonal opening, the diameter of the opening would be the diagonal line of two opposite corners. The dimension of the stop feature is also defined perpendicular to the longitudinal axis of the opening when the fixing element is inserted into the opening. The first stop feature of the fixing element with a dimension greater than a diameter of the opening ensures that the fixing element is limited in its movement along the longitudinal axis of the opening, e.g. during screwing in, by a contact surface of the opening located at its first end. The first end is opposite to its second end which forms the connection with the lumen of the anchor block.

In case the fixing element is a cap screw, the diameter of the head portion and thereby the first stop feature is provided by the maximum radial dimension of the screw head.

The advantage of the lead anchor according to the present invention, wherein the fixing element comprises a first stop feature is that the fixing element during insertion abuts the anchor block at the first end of the opening thus limiting insertion. In this way, it can be ensured, that the fixing element is only inserted into the opening to the extent that the lead is reliably fixed but not damaged by the fixing element. Accordingly, the inventive lead anchor prevents overtightening of the fixing element, e.g. the threaded screw, thereby avoiding over-compression of the lead and thereby damaging of the lead.

Further, the anchor body may comprise a passage, for example a cylindrical passage, which is in line with the lumen of the anchor block. The passage of the anchor body and the lumen of the anchor block may form together a lead-through for a respective neuromodulation lead.

In one embodiment, the head portion comprises at least one second stop feature, wherein said stop feature limits the movement of the fixing element against the direction of insertion. The movement may be limited by abutting of the second stop feature against the anchor body. The fact that the movement of the fixing element is limited means that the second stop feature of the fixing element can only be moved against the direction of insertion up to a predefined maximum length along the longitudinal axis of the opening. It should be noted that the fixing element may be moved further against the direction of insertion but then, however, destroys the anchor body. Further, the second stop feature may also be realized by a flange-like projection or a screw head (cap), for example the same projection or head which forms the first stop feature.

The second stop feature of the head portion of the fixing element may be formed by one or more projections that extends radially from the fastening portion or head. The second stop feature may be, for example, provided by a flange surface arranged at the side of the projection or head pointing away from the fastening portion (i.e. a flange surface of the projection or head that faces away from the fastening portion). The limitation in the movement of the second stop feature may be provided by a flange-like face or step face at an opening within the anchor body, wherein the opening allows access to the head portion of the fixing element for tightening.

By providing a second stop feature on the fixing element, the movement of the fixing element against the direction of insertion can be limited, thus preventing accidental removal of the fixing element.

The first stop feature and the second stop feature may extend partly or fully around the head portion of the fixing element.

In one embodiment the at least one first stop feature and the at least one second stop feature of the fixing element are offset along the longitudinal axis of the fixing element. Thus, having a fixing element comprising two stop features, may also be understood as having a fixing element with one projection, wherein said projection comprises a first contact surface directed towards the fastening portion and a second contact surface pointing towards the opposite direction.

In one embodiment, the lumen provided within the anchor block may be a through hole.

In one embodiment the portion of the neuromodulation lead is at least partially arranged within a sleeve, for example a mesh. Thus, the fixing element being inserted into the opening may engage with the sleeve in order to fix the neuromodulation lead. The sleeve may help to distribute the force applied to the neuromodulation lead over a greater surface of the neuromodulation lead. The sleeve may for example be provided by a compressible mesh or a polymeric sleeve.

In one embodiment, the sleeve is permanently fixed, for example welded, within said lumen of said anchor block. However, the sleeve may also just be inserted into the anchor block and fixed in a different manner. It is important to fix the sleeve inside the anchor block to ensure that the sleeve does not slip when the neuromodulation lead is inserted into the anchor block.

In one embodiment, the anchor block comprises at least one cap arranged at one end of said lumen and configured to secure the sleeve. In the case that the lumen is a cylindrical volume and a longitudinal axis it has two ends along this axis. The cap may be press fitted within said lumen or screwed in said lumen at its respective end. Alternatively, the cap may be fixed within said lumen in an alternative manner, e.g. by welding. By inserting the cap, the diameter of the lumen is reduced so that a previously inserted sleeve is protected from slipping out.

In one embodiment, the anchor block comprises at least one eyelet projecting from the anchor block. The eyelet, i.e. the suture loop, may project in the orthogonal direction compared to the longitudinal extension of the lead anchor. The eyelet may be an integral part of the anchor block or may be attached thereto. Thus, there may be embodiments, wherein the material of the eyelet and the material of the anchor block differ. Albeit, the material of the eyelet comprises a greater material stiffness than the material of the anchor body. In addition, the eyelet may be machined in the same manufacturing process from the same material as the anchor block or may be cast in the same process as the anchor block. Alternatively, the eyelet may be fixedly attached in a joining process like e.g. welding, soldering, brazing. Further, the eyelets may be overmolded by a softer material such as silicone, for example, when the anchor body is produced, but penetrate the material such that they are accessible from the outside. However, independent of the material, the eyelet is always at least configured for securing the lead anchor to the tissue of a patient. For this purpose, the suturing material is guided through the eyelet, wherein grooves may be provided on the anchor body in which the suturing material is guided so that axial slippage of the suturing material in the longitudinal direction of the lead anchor can be prevented.

In one embodiment the at least one eyelet protrudes from the anchor body and penetrates the anchor body. The eyelet may be covered by silicone. The eyelet may emerge from the flexible silicone of the anchor body with the loops exposed for securing the lead anchor to the tissue of a patient. By providing a protruding eyelet, the physician has easier access to the eyelet. At the same time the eyelet is more robust than, for example, silicone eyelets, due to its material stiffness so that the risk of damage or tearing is reduced when the lead anchor is secured to the tissue of the patient.

In one embodiment, the lead anchor comprises at least two eyelets, wherein the eyelets are arranged offset. Providing at least two eyelets helps for a better fixation of the lead anchor in the patient's body providing a distribution of attachment forces. For example, if the eyelets are arranged axially offset along the longitudinal extension of the lead anchor and are laterally offset at the same time, a secure fixation of the lead anchor can be ensured with the help of fewer eyelets.

In one embodiment, the anchor block comprises at least one projection, for example a pin-like projection, projecting perpendicular or inclined from its outer surface, wherein, when the lead anchor is sutured, sewing material is positioned at least partially around the at least one projection. In one embodiment a plurality of projections may be arranged at one end section or both end sections of the anchor block in longitudinal direction of the lumen. The may further be arranged in rows or double rows, wherein the sewing material may be wound around the projection in zigzag. One pin-like projection may comprise a round or angular cross section. The projections may be arranged alone or may be arranged together with the eyelets. In principle the same features of the eyelet mentioned above, apply to the projections so that the projections may be an integral part of the anchor block or attached thereto by common connecting mechanisms. In addition, the at least one projection may penetrate the anchor body such that they are accessible from the outside. To fix the lead anchor in the patient's body, the suture material is placed around the projections in a suitable manner and tied tight. According, the physician is not required to thread suture through a hole but rather thread the suture conveniently around the lead anchor thereby providing a more robust and reliable suture anchoring mechanism. The projections are made, for example, of a metallic material such as titanium alloy or a biocompatible polymer.

In one embodiment the anchor block comprises a metal and/or a polymer material. Further, in one embodiment the anchor body comprises polymer material, for example silicone. As already mentioned above, the anchor block is solid compared to the anchor body, i.e. the material stiffness of the material of the anchor block is greater than the material stiffness of the material of the anchor body. Further, the biocompatibility of the materials should be considered when selecting the materials. Another aspect of the material selection is determined by the material costs and their suitability for production. Therefore, suitable materials for the anchor block may be titanium alloy or a rigid biocompatible polymer such as PEEK.

In one embodiment the head portion of the fixing element comprises a tool mount. The tool mount can be designed to fit with any common tool and may have, for example, a hexagon socket for an Allen key or a cross slot for a screwdriver. The tool mount may extend in longitudinal direction into the fastening portion of the fixing element in order to provide sufficient tool engagement length for the engagement of a tool and the tool mount.

Further, the present invention provides a method for manufacturing the lead anchor, wherein the method comprises the following steps:
- providing the anchor block with the at least one opening;
- inserting the fastening portion of the fixing element into the opening; and
- overmolding the anchor block, leaving at least the head portion of the fixing element partially exposed, thereby creating the anchor body.

It is obvious that depending on the specific design of the embodiment, further steps are needed or may be provided in between the above-mentioned steps. For example, a sleeve may be inserted into the anchor block before overmolding in order to leave the head portion of the fixing element exposed.

By leaving the head portion of the fixing element at least partially exposed and not overmolded, visual feedback is provided to the physician when tightening the fixing element in order to confirm that the tool is fully engaged / seated in the tool mount of the fixing element prior to applying a force (e.g. a torque) to secure the lead. Although the head portion of the fixing element remains partially exposed, the fixing element could be secured from accidental removal by the second stop feature, wherein the stop feature abuts the anchor body. In summary, the anchor block serves to hold the fixing element and to guide a neuromodulation lead. The anchor body surrounds the anchor block and, with its softer material, provides better comfort once the lead anchor is implanted. In addition, the anchor body also serves to guide the neuromodulation lead within its lumen. The fixing element is configured to fix the neuromodulation lead by pressing its end opposite the head portion against the lead or a surrounding sleeve. The fixing element may either abut directly to the neuromodulation lead and/or deform the sleeve to fix the neuromodulation lead. The sleeve thus serves to protect the neuromodulation lead, ensures an even distribution of force and/or fixes the neuromodulation lead. The caps, which may be inserted into the lumen of the lead, secure the sleeve in the lumen. The eyelet or projection helps the physician to fix the lead anchor in the body using suture material.

It will be apparent to those skilled in the art that numerous modifications and variations of the described examples and embodiments are possible in light of the above teaching. The disclosed examples and embodiments are presented for purposes of illustration only. Other alternate embodiments may include some or all of the features disclosed herein. Therefore, it is the intent to cover all such modifications and alternate embodiments as may come within the true scope of this invention, which is defined by the appended claims.

The various features and advantages of the present invention may be more readily understood with reference to the following detailed description and the embodiments shown in the drawings. Herein schematically and exemplarily,
- Fig. 1: shows a top view of a lead anchor according to one embodiment of the invention,
- Fig. 2: depicts a side view of the embodiment of Fig. 1,
- Fig. 3A: shows a longitudinal sectional view of the embodiment of Fig. 1, sectioned along the axis A of Fig. 1,
- Fig. 3B: shows a detailed view of the section B of Fig. 3A,
- Fig. 3C: depicts a detailed center section of Fig. 3A without the fixing element,
- Fig. 4: shows a longitudinal sectional view of the anchor body of the embodiment of Fig. 1 and a corresponding view of the fixing element being separated, and
- Fig. 5: shows a partially transparent view of a second embodiment of the lead anchor.

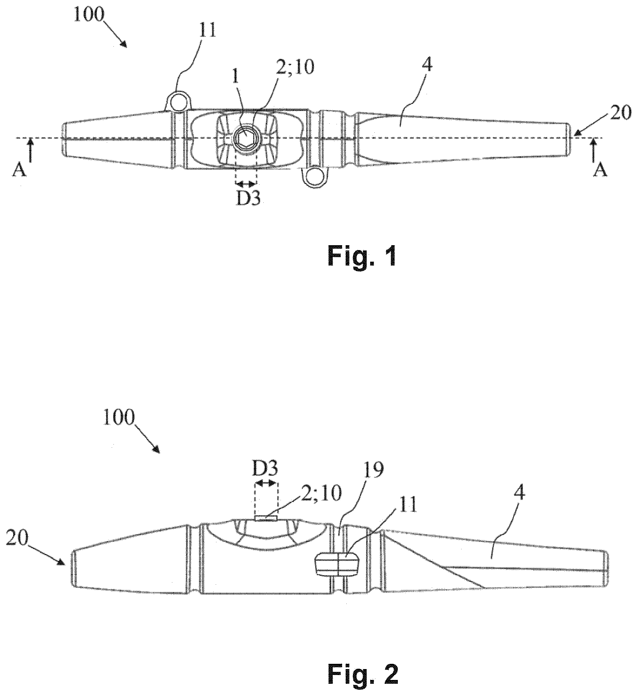

Fig. 1 to 5 show a lead anchor 100 for a neuromodulation lead comprising an anchor block 5, an anchor body 4 and a fixing element 10, wherein the anchor body 4 covers or surrounds the anchor block 5. The fixing element 10 comprises a hexagon socket as a tool mount 1. As Fig. 1 shows a top view of the lead anchor, only a head portion 2 of the fixing element 10 is shown.

Further, two eyelets 11 are provided, wherein the eyelets are axially offset and wherein the eyelets 11 penetrate the anchor body 4. The eyelets 11 are integrally formed with the anchor block 5 and penetrate through the anchor body 4. Fig. 2 shows grooves 19 which may be used to guide the suturing material and thereby prevent axial slippage of the suturing material when the suturing material is fixed at the eyelets 11 during implantation of the lead anchor 100.

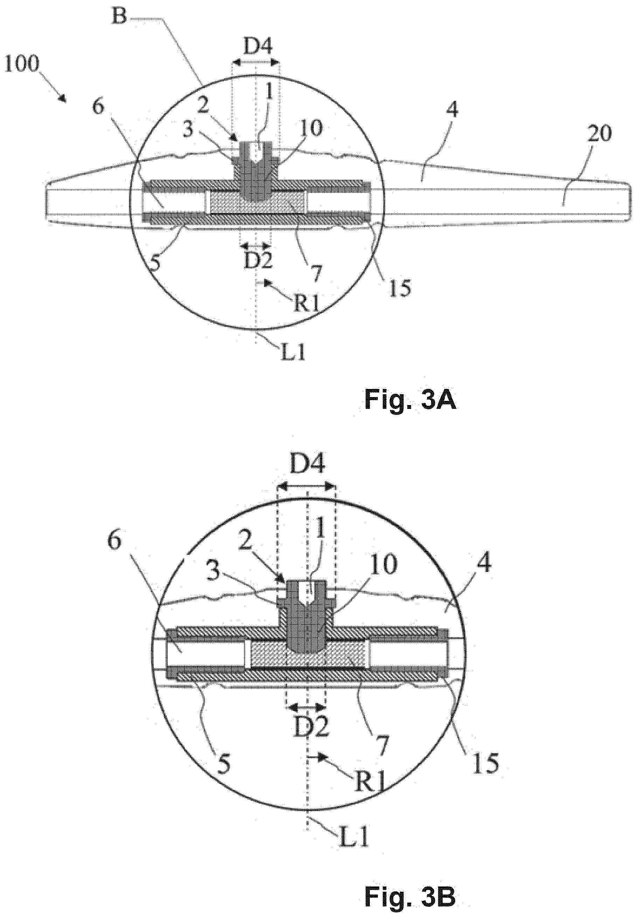

The axis marked with the arrows and letters 'A' represents the cutting axis for the longitudinal sectional views in Figs. 3A and 3B. At the same time the axis extends along the longitudinal extension of the lead anchor 100. Fig. 3A shows that the anchor body 4 comprises a passage 20 which is formed as a cylindrical passage, extending along the cutting axis. The passage 20 is in line with a cylindrical lumen 6 of the anchor block 5 and thereby forming a lead-through for a neuromodulation lead. However, the lumen 6 as well as the passage 20 are best seen in the longitudinal sectional views of Figs. 3A and 3B.

In Fig. 3A it is shown that the anchor block 5 is arranged within the anchor body 4 or the anchor body 4 covers the anchor block 5. Further, the anchor block 5 comprises a sleeve 7 and two press-fitted caps 15 securing said sleeve 7 at both longitudinal ends of lumen 6. Alternatively, those caps 15 may be fixed to the anchor block 5 in a different manner, for example by welding or by screwing. Sleeve 7 may be formed as a mesh.

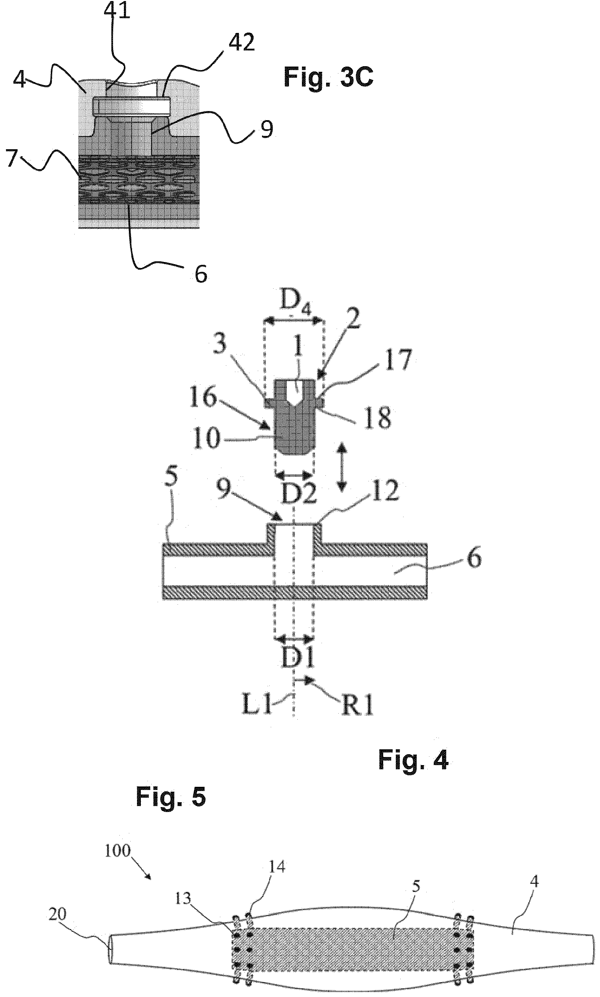

Furthermore, the anchor block 5 comprises a cylindrical opening 9, wherein Fig. 3A and 3B show that the fixing element 10 is already inserted within said opening 9 engaging said sleeve 7. The opening 9 is connected with the lumen 6 and extends perpendicular therefrom. The fixing element 10 comprises the head portion 2 with the tool mount 1 and a washer-like projection 3. The projection 3 forms a flange-like and ring-like lower surface 18 and a flange-like and ring-like upper surface 17, wherein the upper surface 17 is opposite the lower surface 18 (see Fig. 4). The projection 3 and herewith the upper surface 17 and the lower surface 18 have a fourth diameter D4.

The fastening portion 16 (see Fig. 4) extending along the shaft below the projection 8 may comprise a threaded portion, wherein the opening 9 also may provide a threaded portion at its inner surface in order to engage with the fastening portion 16 of the fixing element 10.

A first diameter D1 of the opening 9 of the anchor block 5 is shown in Fig. 4. Further, an arrow with arrowheads on both sides indicates the directions of movement of the fixing element 10 along axis L1, i.e. the direction of insertion and the direction against insertion. In addition, a contact surface 12 at a first end of the opening 9 is shown.

As the diameter D4 of the lower surface 18 of the fixing element 10 is greater than the diameter D1 of the opening 9, the lower surface 18 abuts to the contact surface 12 of the opening 9 when the fixing element 10 is inserted into the opening thereby limiting its movement into the direction of lumen 6.

As shown in Fig. 3C the anchor body 4 comprises an opening 41, as well, having a diameter D3 (see Fig. 1 and 2). A widened portion of this opening 41 located just over the contact surface 12 of opening 9 of the anchor block 5, terminates in upper direction (i.e. away from the opening 9 and the lumen 6) with a flange-like and ring-like surface 42. As the diameter D4 of the upper surface 17 of the fixing element 10 is greater than the diameter D3, the upper surface 17 abuts to the flange-like surface 42 of the anchor body 4 when the fixing element 10 moves in a direction away from the lumen 6 thereby avoiding accidental removal of the fixing element 10.

Accordingly, the lower surface 18 forms the first stop feature according to the invention and the upper surface 17 forms the second stop feature which are provided by the same projection 3 but are offset along the longitudinal direction L1.

The second diameter D2 is the diameter of the fastening portion of the fixing element 10 which is slightly less than the first diameter D1 of opening 9.

Summarizing, the lead anchor 100 according to the first embodiment is formed by the anchor block 5 with the lumen 6 and the opening 9, and the eyelets 11 projecting from the anchor block 5. The opening 9 is provided with corresponding engagement means, so that the fixing element 10 can engage with the opening 9. If necessary, the sleeve 7 is provided within the lumen 6 of the anchor block 5, wherein the sleeve 7 may be welded into the anchor block 5, held by caps 15 or fixed in the anchor block 5 in some other way.

In Fig. 5 a lead anchor 100 of a second embodiment is shown in a top view, with the anchor body 4 being partially transparent, so that the anchor block 5 is visible inside the anchor body 4. Instead of eyelets, Fig. 5 has four rows of projections 13, wherein each two rows are located at the right and left end section of anchor block 5 in longitudinal direction. In total, the lead anchor 100 shows six projections 13 per side. The pin-like projections project perpendicularly from the outer surface of the anchor block 5 and protrude through the anchor body 4 so that they are accessible from the outside. Alternatively, one row of projections may be provided at each end section.

The lead anchor shown in Fig. 5 comprises four rings 14, one ring 14 per row of projections 13. Alternatively, there can be more or less projections 13 and correspondingly more or less rings 14. The projections 13 help a physician to suture the lead anchor 100 in the patient's body, for example in his or her fascia. The suturing material can be guided around the projections 13 in a suitable manner, so that the lead anchor 100 can be held in place. Further, the suturing material may be guided through the rings 14. Thus, the rings 14 may prevent the suturing material from slipping axially in the longitudinal direction of the lead anchor 100.

Even if no further embodiments are shown, the different components of the different embodiments of the present invention can of course be combined with each other as desired.

Hereinafter, during manufacturing of the lead anchor (for example a lead anchor 100 of the first embodiment) the anchor block 5 is provided and the fixing element 10 is inserted and screwed into the opening 9 along the longitudinal axis L1 until the first stop feature 18 abuts the contact surface 12 of the anchor block 5. It is then screwed back for a small distance such that it does not abut the contact surface 12 anymore. The anchor block 5 is then overmolded with a soft material such as silicone, leaving part of the head portion 2 of the fixing element 10 exposed by forming the opening 41 and the flange surface 42 of the anchor body 4. The overmolding material forms the anchor body 4 which comprises the passage 20.

With the fixing element 10 is partially loosened, i.e. its projection 3 having a short distance to the contact surface 12 of opening 9 of the anchor block 5 a neuromodulation lead (not shown) is passed through the lead anchor 100, i.e. through the lead-through formed by the lumen 6 and the passage 20. The fixing element 10 may then be used to fix the neuromodulation lead by screwing the fixing element 10 into the opening 9 using the tool mount 1 until the first stop feature (lower surface 18) of the fixing element 10 abuts the contact surface 12 of the anchor block 5, thereby limiting the movement of the fixing element 10 into the insertion direction, and thus limiting the force to be applied on the neuromodulation lead. The lead anchor 100 is then sutured into the patient body, wherein the suturing material is guided through the eyelets 11 and/or fixed with the help of the projections 13. In addition, the grooves 19 may be used to guide the suturing material.

The individual steps of a possible application of the lead anchor 100 described above should not be understood as limiting. Just as it may be possible that the fixing element 10 is inserted into the anker block 5 after overmolding the anchor block 5, it is possible that the lead anchor 100 is first sutured into the patient's body and then the neuromodulation lead is guided through the lead anchor 100 and fixed in place with the fixing element 10.

### Reference numerals

- 1: Tool mount
- 2: Head portion of fixing element 10
- 3: Projection
- 4: Anchor body
- 5: Anchor block
- 6: Lumen
- 7: Sleeve
- 9: Opening
- 10: Fixing element
- 11: Eyelet
- 12: Contact surface of the opening 9
- 13: Projection
- 14: Ring
- 15: Cap
- 16: Fastening portion of fixing element 10
- 17: upper surface (second stop feature)
- 18: lower surface (first stop feature)
- 19: Groove
- 20: Lead-through
- 100: Lead anchor
- D1: Fist diameter (of the opening)
- D2: Second diameter (of the fastening portion)
- D3: Third diameter (of the opening 41 of the anchor body 4)
- D4: Fourth diameter (of the projection 3)
- L1: Longitudinal axis
- R1: Radial direction

## Claims

1. A lead anchor (100) for a neuromodulation lead comprising:
a solid anchor block (5) having a lumen (6) and at least one opening (9), wherein said lumen (6) is configured to receive a portion of the neuromodulation lead and wherein said opening (9) is connected with said lumen (6) and has a longitudinal axis (L1);
an anchor body (4) at least partially covering said anchor block (5); and
a fixing element (10) having a fastening portion (16) and a head portion (2), wherein said fastening portion (16) is configured to be inserted along the axis (L1) into said opening (9) to fix the portion of the neuromodulation lead, wherein said head portion (2) comprises at least one first stop feature (18) having a dimension (D4) in a radial direction (R1) with respect to said axis (L1) which is greater than a diameter (D1) of said opening (9);
**characterized in that**
the anchor block (5) comprises at least one projection (13) or at least one eyelet (11) projecting from the anchor block (5),
wherein the at least one projection (13) or the at least one eyelet (11) protrudes from the anchor body (4) and penetrates the anchor body (4).

2. The lead anchor (100) according to claim 1, wherein the head portion (2) comprises at least one second stop feature (17), wherein said stop feature (17) limits the movement of the fixing element (10) against the direction of insertion.

3. The lead anchor (100) according to claim 2, wherein the at least one first stop feature (18) and the at least one second stop feature (17) of the fixing element (10) are offset along the longitudinal extension of the fixing element (10) along the axis (L1).

4. The lead anchor (100) according to any of the preceding claims, wherein the portion of the neuromodulation lead is at least partially arranged within a sleeve (7), for example a mesh.

5. The lead anchor (100) according to claim 4, wherein the sleeve (7) is permanently fixed, for example welded, within said lumen (6) of said anchor block (5).

6. The lead anchor (100) according to claim 4, wherein the anchor block (5) comprises at least one cap (15) arranged at one end of said lumen (6) and configured to secure the sleeve (7).

7. The lead anchor (100) according to any of the preceding claims, comprising at least two eyelets (11), wherein the eyelets (11) are arranged offset.

8. The lead anchor (100) according to any of the preceding claims, wherein the anchor block (5) comprises at least one projection (13), wherein, when the lead anchor (100) is sutured, sewing material is positioned at least partially around the at least one projection (13).

9. The lead anchor (100) according to any of the preceding claims, wherein the anchor block (5) comprises a metal and/or a polymer material.

10. The lead anchor (100) according to any of the preceding claims, wherein the anchor body (4) comprises polymer material, for example silicone.

11. The lead anchor (100) according to any of the preceding claims, wherein the head portion (2) of the fixing element (10) comprises a tool mount (1).

12. A method for manufacturing the lead anchor (100) according to any of the preceding claims, wherein the method comprises the following steps:
• providing the anchor block (5) with the at least one opening (9);
• inserting the fixing element (10) with its fastening portion (16) into the opening (9); and
• overmolding the anchor block (5), leaving at least the head portion (2) of the fixing element (10) partially exposed, thereby creating the anchor body (4).

## Patentansprüche

1. Elektrodenverankerung (100) für eine Neuromodulationselektrodenleitung, umfassend:
einen festen Ankerblock (5) mit einem Lumen (6) und mindestens einer Öffnung (9), wobei das Lumen (6) dazu eingerichtet ist, einen Abschnitt der Neuromodulationselektrodenleitung aufzunehmen, und wobei die Öffnung (9) mit dem Lumen (6) verbunden ist und eine Längsachse (L1) aufweist;
einen Ankerkörper (4), der den Ankerblock (5) zumindest zum Teil bedeckt; und
ein Fixierelement (10), das einen Befestigungsabschnitt (16) und einen Kopfabschnitt (2) aufweist, wobei der Befestigungsabschnitt (16) dazu eingerichtet ist, entlang der Achse (L1) in die Öffnung (9) eingeführt zu werden, um den Abschnitt der Neuromodulationselektrodenleitung zu fixieren, wobei der Kopfabschnitt (2) mindestens ein erstes Anschlagmerkmal (18) aufweist, das in einer in Bezug auf die Achse (L1) radialen Richtung (R1) eine Abmessung (D4) aufweist, die größer ist als ein Durchmesser (D1) der Öffnung (9);
**dadurch gekennzeichnet, dass**
der Ankerblock (5) mindestens einen Vorsprung (13) oder mindestens eine Durchführung (11) umfasst, der bzw. die von dem Ankerblock (5) vorsteht,
wobei der mindestens eine Vorsprung (13) oder die mindestens eine Durchführung (11) von dem Ankerkörper (4) vorsteht und durch den Ankerkörper (4) hindurch geht.

2. Elektrodenverankerung (100) nach Anspruch 1, wobei der Kopfabschnitt (2) mindestens ein zweites Anschlagmerkmal (17) umfasst, wobei das Anschlagmerkmal (17) die Bewegung des Fixierelements (10) entgegen der Einführrichtung begrenzt.

3. Elektrodenverankerung (100) nach Anspruch 2, wobei das mindestens eine erste Anschlagmerkmal (18) und das mindestens eine zweite Anschlagmerkmal (17) des Fixierelements (10) entlang des longitudinalen Verlaufs des Fixierelements (10) entlang der Achse (L1) versetzt sind.

4. Elektrodenverankerung (100) nach einem der vorangehenden Ansprüche, wobei der Abschnitt der Neuromodulationselektrodenleitung zumindest zum Teil innerhalb einer Hülse (7), beispielsweise eines Gitters, angeordnet ist.

5. Elektrodenverankerung (100) nach Anspruch 4, wobei die Hülse (7) dauerhaft innerhalb des Lumens (6) des Ankerblocks (5) befestigt, zum Beispiel angeschweißt, ist.

6. Elektrodenverankerung (100) nach Anspruch 4, wobei der Ankerblock (5) mindestens eine Kappe (15) umfasst, die an einem Ende des Lumens (6) angeordnet und dazu eingerichtet ist, die Hülse (7) zu sichern.

7. Elektrodenverankerung (100) nach einem der vorangehenden Ansprüche, mindestens zwei Durchführungen (11) umfassend, wobei die Durchführungen (11) versetzt angeordnet sind.

8. Elektrodenverankerung (100) nach einem der vorangehenden Ansprüche, wobei der Ankerblock (5) mindestens einen Vorsprung (13) umfasst, wobei beim Nähen der Elektrodenverankerung (100) Nähmaterial zumindest zum Teil um den mindestens einen Vorsprung (13) herum positioniert wird.

9. Elektrodenverankerung (100) nach einem der vorangehenden Ansprüche, wobei der Ankerblock (5) ein Metall- und/oder ein Polymermaterial umfasst.

10. Elektrodenverankerung (100) nach einem der vorangehenden Ansprüche, wobei der Ankerblock (4) Polymermaterial, zum Beispiel Silikon, umfasst.

11. Elektrodenverankerung (100) nach einem der vorangehenden Ansprüche, wobei der Kopfabschnitt (2) des Fixierelements (10) eine Werkzeugaufnahme (1) umfasst.

12. Verfahren zum Herstellen der Elektrodenverankerung (100) nach einem der vorangehenden Ansprüche, wobei das Verfahren die folgenden Schritte umfasst:
• Bereitstellen des Ankerblocks (5) mit der mindestens einen Öffnung (9);
• Einführen des Fixierelements (10) mit seinem Befestigungsabschnitt (16) in die Öffnung (9); und
• Ausbilden des Ankerblocks (5) durch Umspritzung, wobei zumindest der Kopfabschnitt (2) des Fixierelements (10) teilweise frei gelassen wird, um dadurch den Ankerkörper (4) zu erzeugen.

## Revendications

1. Dispositif d'ancrage de câble (100) destiné à un câble de neuromodulation comprenant :
un bloc d'ancrage solide (5) ayant une lumière (6) et au moins une ouverture (9), dans lequel ladite lumière (6) est conçue pour recevoir une partie du câble de neuromodulation et dans lequel ladite ouverture (9) est reliée à ladite lumière (6) et a un axe longitudinal (L1) ;
un corps d'ancrage (4) recouvrant au moins en parti ledit bloc d'ancrage (5) ; et
un élément de fixation (10) ayant une partie d'attache (16) et une partie de tête (2), dans lequel ladite partie d'attache (16) est conçue pour être insérée le long de l'axe (L1) dans ladite ouverture (9) afin de fixer la partie du câble de neuromodulation, dans lequel ladite partie de tête (2) comprend au moins une première caractéristique d'arrêt (18) ayant une dimension (D4) dans une direction radiale (R1) par rapport audit axe (L1) qui est supérieure à un diamètre (D1) de ladite ouverture (9) ;
**caractérisé en ce que**
le bloc d'ancrage (5) comprend au moins une saillie (13) ou au moins un œillet (11) faisant saillie depuis le bloc d'ancrage (5),
dans lequel l'au moins une saillie (13) et l'au moins un œillet (11) fait saillie depuis le corps d'ancrage (4) et pénètre le corps d'ancrage (4).

2. Dispositif d'ancrage de câble (100) selon la revendication 1, dans lequel la partie de tête (2) comprend au moins une seconde caractéristique d'arrêt (17), dans lequel ladite caractéristique d'arrêt (17) limite le mouvement de l'élément de fixation (10) vers le sens d'insertion.

3. Dispositif d'ancrage de câble (100) selon la revendication 2, dans lequel l'au moins une première caractéristique d'arrêt (18) et l'au moins une seconde caractéristique d'arrêt (17) de l'élément de fixation (10) sont décalées le long de l'extension longitudinale de l'élément de fixation (10) le long de l'axe (L1).

4. Dispositif d'ancrage de câble (100) selon l'une quelconque des revendications précédentes, dans lequel la partie du câble de neuromodulation est au moins partiellement agencée au sein d'un manchon (7), par exemple une maille.

5. Dispositif d'ancrage de câble (100) selon la revendication 4, dans lequel le manchon (7) est fixé de manière permanente, par exemple soudé, à l'intérieur de ladite lumière (6) dudit bloc d'ancrage (5).

6. Dispositif d'ancrage de câble (100) selon la revendication 4, dans lequel le bloc d'ancrage (5) comprend au moins un capuchon (15) agencé à une extrémité de ladite lumière (6) et conçu pour verrouiller le manchon (7).

7. Dispositif d'ancrage de câble (100) selon l'une quelconque des revendications précédentes, comprenant au moins deux œillets (11), dans lequel les œillets (11) sont agencés de manière décalée.

8. Dispositif d'ancrage de câble (100) selon l'une quelconque des revendications précédentes, dans lequel le bloc d'ancrage (5) comprend au moins une saillie (13), dans lequel, lorsque le dispositif d'ancrage de câble (100) est suturé, le matériel de couture est positionné au moins partiellement autour de l'au moins une saillie (13).

9. Dispositif d'ancrage de câble (100) selon l'une quelconque des revendications précédentes, dans lequel le bloc d'ancrage (5) comprend un métal et/ou un matériau polymère.

10. Dispositif d'ancrage de câble (100) selon l'une quelconque des revendications précédentes, dans lequel le bloc d'ancrage (4) comprend un matériau polymère, par exemple la silicone.

11. Dispositif d'ancrage de câble (100) selon l'une quelconque des revendications précédentes, dans lequel la partie de tête (2) de l'élément de fixation (10) comprend un logement d'outil (1).

12. Procédé de fabrication du dispositif d'ancrage de câble (100) selon l'une quelconque des revendications précédentes, dans lequel le procédé comprend les étapes suivantes :
• pourvoir le bloc d'ancrage (5) avec l'au moins une ouverture (9) ;
• insérer l'élément de fixation (10) avec sa partie d'attache (16) dans l'ouverture (9) ; et
• surmouler le bloc d'ancrage (5), laissant au moins la partie de tête (2) de l'élément de fixation (10) partiellement exposée, créant ainsi le corps d'ancrage (4).
